(19) Europäisches Patentamt
European Patent Office
Office européen des brevets

(11) Publication number: **0 221 208 B1**

(12) **EUROPEAN PATENT SPECIFICATION**

(45) Date of publication of patent specification: **25.09.91**

(51) Int. Cl.[5]: **A61K 7/06**, C07K 7/06, C07K 9/00

(21) Application number: **85306970.6**

(22) Date of filing: **30.09.85**

(54) **Hair growth promoting compositions.**

(43) Date of publication of application:
**13.05.87 Bulletin 87/20**

(45) Publication of the grant of the patent:
**25.09.91 Bulletin 91/39**

(84) Designated Contracting States:
**DE FR GB IT**

(56) References cited:
**EP-A- 0 082 568**

**CHEMICAL ABSTRACTS, vol. 90, 1979, page 669, ref.no. 20405m; Columbus, Ohio US & JP-A-78147093 (FUNAI PHARMACEUTICAL INC. Ltd.) 21-12-1978 * Abstract ***

**CHEMICAL ABSTRACTS, vol.91, 1979, page 717, ref.no. 21126q; Columbus, Ohio, US & JP-A-78147092 ( funai pharmaceutical ind. Ltd.) 21.12.1978 * Abstract ***

**CHEMICAL ABSTRACTS, vol. 90, 1979, page 669, ref.no. 204506n; Columbus, Ohio, US & JP-A-78147064 (FUNAI PHARMACEUTICAL IND. Ltd.) 21-12-1978. * Abstract ***

(73) Proprietor: **MEIJI SEIKA KAISHA LTD.**
**4-16 Kyobashi 2-chome**
**Chuo-ku Tokyo 104(JP)**

(72) Inventor: **Matsuo, Yutaka**
**5-17-6, Roppongi**
**Minato-ku Tokyo(JP)**
Inventor: **Yanaihara, Noboru**
**403-22, Kita**
**Shizuoka-shi Shizuoka-ken(JP)**
Inventor: **Watanabe, Suzuo**
**2-15-4, Iwato**
**Yokosuka-shi Kanagawa-ken(JP)**
Inventor: **Sato, Toyomi**
**4-22-10, Higashi Nippori**
**Arakawa-ku Tokyo(JP)**
Inventor: **Kikkoji, Toshihiro**
**1-11-12, Sanno**
**Ohta-ku Tokyo(JP)**
Inventor: **Kawashima, Junichi**
**2-9-22, Himonya**
**Meguro-ku Tokyo(JP)**

**EP 0 221 208 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

CHEMICAL ABSTRACTS, vol. 90, 1979, page 704, ref. no. 187352q; Columbus, Ohio, US & JP-A- 78147065 (FUNAI PHARMACEUTICAL IND. Ltd.) 21.12.1978 * Abstract *

THE MERCK INDEX, 10th edition, 1983, ref. no. 8738: " Substance P"; Merck & Co, INc., Rahway, N.J., US * Abstract *

CHEMICAL ABSTRACTS, vol. 104, 1986, page 429, ref.no. 95263r; Columbus, Ohio, US &JP-A-60202807 ( Meiji Seika Kaisha Ltd.) 14.10.1985 * Abstract *

Inventor: Kadosawa, Hiroyuki
107-13, Tomuro
Atsugi-shi Kanagawa-ken(JP)
Inventor: Hirano, Fumiya
515-1, Shinano-machi Totsuka-ku
Yokohama-shi Kanagawa-ken(JP)

(74) Representative: Moon, Donald Keith et al
BREWER & SON Quality House Quality Court
Chancery Lane
London WC2A 1HT(GB)

## Description

This invention relates to new, hair-growth-promoting compositions which activate the hair matrix cells, promote the growth of the hair and prevent the falling-out of the hair from scalp, and which comprises Substance P or a Substance P-related peptide as a principal, active ingredient for the promotion of the hair growth.

Various kinds of promoter for the growth of the hair in the scalp have been used for the cosmetic purpose. While, it is known that Substance P is a known undecapeptide having eleven amino acid residues in its molecule and represented by the formula

$H-Arg-Pro-Lys-Pro-Gln-Gln-Phe-Phe-Gly-Leu-Met-NH_2$ (II)

wherein Arg stands for arginine residue, Pro stands for proline residue, Lys stands for lysine residue, Gln stands for glutamine residue, Phe stands for phenylalanine residue, Gly stands for glycine residue, Leu stands for the leucine residue Met stands for methionine residue according to the normal abbreviation of amino acids (see the "J. Physiol." 72, 74 (1931); the "J.Physiol." 79, 255 (1933); "Substance P", edited by von Euler, U.S. & Pernow, B, Raven Press, New York, 1977; and a Japanese literature "I-gaku-no A yumi" Vol. 101, No. 4, pages 179-185, published 23rd July, 1977).

Substance P is a physiologically active peptide which was discovered several years ago to be a neurotransmitter for the neurone of the centripetal, sensory nerves, and different physiological activities of Substance P have been studied in the recent years. It is known that Substance P exhibits physiological activities or pharmacological activities, including an ileum-contracting activity and a blood-pressure-reducing activity. Besides, some derivatives of Substance P, termed as Substance P-related peptides, which exhibit much improved physiological and pharmacological properties than Substance P have been synthesized, and it has been confirmed that these Substance P-related peptides have a remarkable ileum-contracting activity and a remarkable hypertensive activity (see the above-mentioned Japanese literature "I-gaku-no-A-yumi" and Japanese patent application un-examined first publication "Kokai" Nos. 147064/78; 147065/78; 147092/78 and 147093/78).

We, the present inventors, have made researches and have now found that Substance P and some Substance P-related peptides represented by the general formula (I)

$X-Phe-Phe-Gly-Leu-Met-NH_2$ (I)

wherein Phe, Gly, Leu and Met have the same meanings as defined above, respectively, and X stands for pyro-glutamic acid residue (abbreviated as pGlu) or a peptide composed of 2 to 5 amino acid residues or a nucleoside exhibit an activity of increasing the blood flow in the blood vessels below the skin surface when an effective amount of Substance P or a Substance P-related peptide of the formula (I) has been applied externally to the skin surface, and that Substance P and a Substance P-related peptide of the formula (I) are highly effective to activate the hair matrix cells and promote the growth of the hair. We have also found that the effect for promotion of the hair growth may be obtained reproducibly by repeated applications of certain Substance P or Substance P-related peptide containing compositions.

According to the first aspect of this invention, therefore, there is provided a hair-growth-promoting composition comprising (a) as the active ingredient an effective amount of at least one of Substance P and a Substance P-related peptide of the formula (I)

$X-Phe-Phe-Gly-Leu-Met-NH_2$ (I)

wherein Phe denotes the phenylalanine reside, Gly denotes the glycine residue, Leu denotes the leucine reside, Met denotes the methionine residue and X denotes the pyroglutamic acid residue (pGlu) or a peptide composed of 2 to 5 amino acid residues or a nucleoside, or an acid addition salt thereof, and (b) at least one of higher aliphatic alcohols and carboxyvinyl polymers which are effective as an agent for controlling the absorption of the active peptide by the skin, in association with (c) a known vehicle or excipient for the active peptide (a) and the agent (b) for controlling the absorption of the active peptide.

Particular examples of the Substance P-related peptide of the general formula (I) include such derivatives of Substance P as listed in Table 1 below.

Table 1

| Formula of Peptides | Abbreviation |
|---|---|
| H-Gln-Phe-Phe-Gly-Leu-Met-$NH_2$ | Substance $P_{6-11}$ |
| H-Gln-Gln-Phe-Phe-Gly-Leu-Met-$NH_2$ | Substance $P_{5-11}$ |
| H-Pro-Gln-Gln-Phe-Phe-Gly-Leu-Met-$NH_2$ | Substance $P_{4-11}$ |
| H-Lys-Pro-Gln-Gln-Phe-Phe-Gly-Leu-Met-$NH_2$ | Substance $P_{3-11}$ |
| H-Pro-Lys-Pro-Gln-Gln-Phe-Phe-Gly-Leu-Met-$NH_2$ | Substance $P_{2-11}$ |
| pGlu-Phe-Phe-Gly-Leu-Met-$NH_2$ | p-Glu-Substance $P_{7-11}$ |
| pGlu-Gln-Phe-Phe-Gly-Leu-Met-$NH_2$ | p-Glu-Substance $P_{6-11}$ |
| 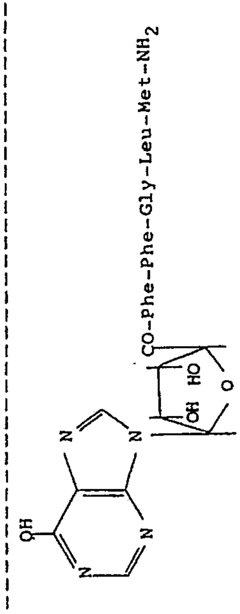 | Substance MH-548 |
| 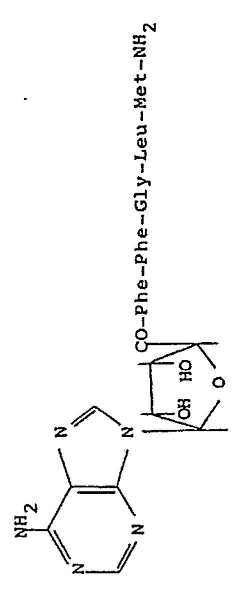 | Substance MH-648 |

In the composition according to this invention, the Substance P or Substance P-related peptide of the formula (I) may be in the form of its acid addition salt with a pharmaceutically acceptable inorganic acid such as hydrochloric acid, sulfuric acid, phosphoric acid or with a pharmaceutically acceptable organic acid such as acetic acid, propionic acid and citric acid.

In the composition according to this invention, the vehicle for the active ingredient peptide may be such an appropriate liquid or solid or semi-solid vehicle or carrier which is conventionally used in the known

cosmetic preparations or the known hair-growth-promoting compositions. The vehicle available in the composition of this invention includes, for example, water, ethanol, aqueous ethanol, a usual creamy excipient and a usual oily excipient or substrate known in the art of cosmetics.

Thus, we have observed that when Substance P of the formula (II) or a Substance P-related peptide of the formula (I) is applied externally to the skin of scalp or other parts of the human body, Substance P or the Substance P-related peptide is absorbed into the skin layer to increase the blood flow under the local region of the skin surface as treated with Substance P or Substance P-related peptide, leading to activation of the hair matrix cells and promotion of the hair growth as well as prevention or reduction of the falling-out of the hair. Through our further study, however, we have now found also that the biological reactions as caused by the Substance P or Substance P-related peptide as absorbed in vivo are lasting only in a very short time and are thus only transitory, probably because the Substance P or the Substance P-related peptide as absorbed could be degraded and inactivated fastly by peptidases in vivo.

Accordingly, we have found that repeated applications of the active peptide of the formula (I) or (II) to the skin are disadvantageously needed in order to assure achievement of the significant effects of activation of the hair matrix cells and promotion of the hair growth as well as of prevention of the falling-out of the hair, as far as the active peptide of the formula (I) or (II) is used singly.

Generally, the active peptide of the formula (I) of (II) for promotion of the hair growth may be given into the true skin where the hair roots are present, through one of different routes of administration, for instance, by subcutaneous injection or by intravenous injection or even by oral administration. In case the active peptide of the formula (I) or (II) is administered by subcutaneous injection, intravenous injection or orally, however, the active peptide as administered can undesirably be degraded and inactivated at a high rate under the action of the peptidases in vivo, before the active peptide reaches the sites of the hair roots. On the other hand, when the active peptide as specified above is externally applied directly to the skin surface, the active peptide can be absorbed immediately into the skin surface and penetrate into the underlying true skin layer, with diffusing in the true skin to reach the hair roots, so that the active peptide can be degraded and inactivated to a lesser extent in the true skin layer. Besides, external application of the active peptide is obviously a most facile way of administration of the peptide and hence should be a most ready route of administration of the active peptide for the purpose of promoting the hair growth. We have presumed that if portions of the active peptide of the formula (I) or (II) as once applied externally to the skin surface can be arranged to be supplied continuously in a sustained way into the underlying true skin layer for a prolonged time, they are able to compensate for and supplement the quantity of the active peptide just degraded in vivo and maintain an increased blood flow once induced by said peptide under the skin surface for a prolonged time, leading to a meritable consequence such that the effects of the active peptide of promoting the hair growth are further enhanced. Thus, with this presumption, we have made our extensive researches in an attempt to devise and prepare such a formulation comprising the hair growth promotor, which can assure that a coat of the hair-growth-promoting formulation containing as the principal, active ingredient the peptide of the formula (I) or (II) as applied externally onto the skin surface is able to afford and supply the active peptide in portions from said coat into the skin surface and also into the skin layer continuously at a controlled rate and in a sustained way for a prolonged time and is thus able to adjust or control the quantity or rate of said active peptide to be actually absorbed into the skin; and which can also assure that the active peptide once absorbed in the skin penetrates and diffuses into the underlying true skin layer at an increased rate.

As a result of our researches, we have now found that when an active peptide of the formula (I) or (II) as the active ingredient for promotion of the hair growth is incorporated, together with one or more of higher aliphatic alcohols and carboxyvinyl polymers, into a known aqueous or ethanolic excipient conventionally used for the hair-growth promotors of the prior art, such as water and aqueous ethanol, there is prepared such an emulsion-like or emulsifiable lotion or tonic formulation in which the active peptide has been dissolved or suspended in the excipient medium; and that when such lotion or tonic type formulation is applied to the skin surface, the skin surface is covered with a coat of the said formulation from which the active peptide effective to promote the hair growth is gradually released in a sustained way and supplied continuously but at a controlled rate into the skin surface, so that the active peptide as released from the hair-growth-promoting formulation is controlled to be absorbed by the skin, and in other words, the said hair-growth-promoting formulation is able to act like a so-called "sustained release drug" of the hair-growth-promoting peptide. Thus, we have discovered that the higher aliphatic alcohols or the carboxyvinyl polymers, when associated with the hair-growth-promoting active peptide of the formula (I) or (II), can serve as an agent for controlling the absorption of said active peptide by the skin.

Furthermore, we have now found that when one or more of lower alkanols, glycols and some kinds of carboxylic acid esters is or are further added into the hair-growth-promoting formulation prepared as above,

the effects of promotion of the hair growth can be enhanced remarkedly much than in the case where the addition of the lower alkanols, glycols and carboxylic acid esters is not made. Thus, we have discovered that the lower alkanols, glycols and some kinds of carboxylic acid esters can serve as an agent for promoting that the active peptide of the formula (I) or (II) as once absorbed in the skin surface penetrates and diffuses into the true skin layer to reach the hair roots.

Examples of the higher aliphatic alcohols which are effective as the above-mentioned agent for controlling the absorption of the active peptide by the skin include cetanol, lauryl alcohol, stearyl alcohol, oleyl alcohol and lanolic alcohol.

The carboxyvinyl polymer which is available according to this invention as said agent for controlling the absorption of the active peptide by the skin may be such a high-molecular, acidic polymer mainly comprising acrylic acid as the monomeric unit and having an average molecular weight ranging from 1,000,000 to 3,000,000. The carboxyvinyl polymer may preferably exhibit its properties suitable for use in cosmetics and can meet the standard requirements for the materials for use in cosmetic products, for example, in respect of the particular properties as set forth in the Japanese document "Kesho-shin Genryo Kijun" (English transliterated title: Standards for Cosmetic Materials) (published from Yakuji Nippon Sha, Japan). Suitable examples of the carboxyvinyl polymer available in this invention for the above-mentioned purpose are such high molecular-weight, colloidally water-soluble and acidic polymers of acrylic acid cross-linked with approximately 1% by weight of a poly-allyl sucrose where the sucrose molecule has an average of about 5.8 allyl groups per molecule, which are described in U.S. Patent Nos. 2,798,053; 2,985,625; 2,909,462; 2,912,358; 3,027,303; 3,133,865; 3,277,013 and 3,592,936. A preferred example of the carboxyvinyl polymer available in this invention is such a high molecular-weight, colloidally water-soluble and acidic polymer of acrylic acid crosslinked with a polyallyl sucrose as sold under the tradename "HIVIS WAKO-150" (a product of a Japanese company, Wako Junyaku Kogyo Co., Ltd.) which is acidic in nature and in the form of a colloidally water-soluble powder having the gel-forming qualities and having a bulk density of 0.18 to 0.2 g/ml, which gives a thixotropic, clear and viscous solution when dissolved in water and which is believed to be a high molecular-weight and colloidally water-soluble polymer of acrylic acid crosslinked with approximately 1% by weight of a poly-allyl sucrose where the sucrose molecule has an average of about 5.8 allyl group per molecule, alrthrough the precise composition of this resin is not directly revealed by the manufacturer of this resin. The above acrylic acid polymer may preferably be present in the composition of this invention in such a form that the acrylic acid polymer has at least partially been neutralized with sodium hydroxide.

According to a second aspect of this invention, there is provided a hair-growth-promoting composition comprising (a) an effective amount of at least one of Substance P and a Substance P-related peptide of the formula (I)

X-Phe-Phe-Gly-Leu-Met-$NH_2$ (I)

wherein Phe denotes the phenylalanine residue, Gly the glycine residue, Leu the leucine residue, Met the methionine residue, and X denotes the pyro-glutamic acid residue (pGlu) or a peptide composed of 2 to 5 amino acid residues or a nucleoside, or an acid addition salt thereof as the active ingredient for promotion of the hair growth, (b) at least one of higher aliphatic alcohols and carboxyvinyl polymers which are effective as an agent for controlling the absorption of the active peptide by the skin, and (d) at least one of lower alkanols, glycols, mono-esters of a mono-carboxylic acid of 9 to 14 carbon atoms with an alkanol of 1 to 6 carbon atoms, and di-esters of a dicarboxylic acid of 6 to 10 carbon atoms with an alkanol of 1 to 4 carbon atoms which are effective as an agent for promoting the penetration of the active peptide into the true skin, in association with (c) a known vehicle or excipient for the active peptide (a) and the agent (b) for controlling the absorption of the active peptide as well as (c) the agent for promoting the penetration of the active peptide.

In the compositions of this invention, the Substance P-related peptide of the formula (II) is preferably such one of the formula (II) where X denotes a glutamine residue (H-Gln-), a pyro-glutamic acid residue (pGlu-), a dipeptide residue composed of two glutamine molecules (H-Gln-Gln-), a tri-peptide residue composed of two glutamine molecules and one terminal proline molecule (H-Pro-Gln-Gln-), a tetra-peptide residue composed of two glutamine molecules, one proline molecule and one terminal lysine molecule (H-Lys-Pro-Gln-Gln-),a penta-peptide reside composed of two glutamine molecules, one proline molecule, one lysine molecule and one terminal proline molecule (H-Pro-Lys-Pro-Gln-Gln-), or a nucleoside residue of the formula

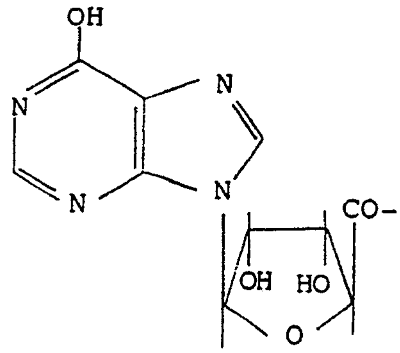

or a nucleoside residue of the formula

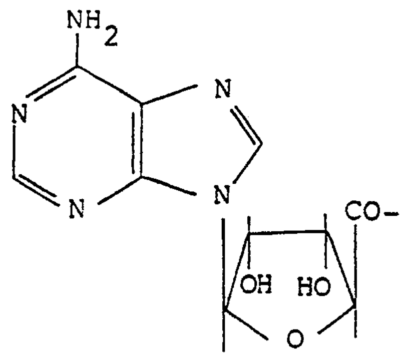

Among the Substance P-related peptides of the formula (I), Substance $P_{6-11}$, Substance $P_{4-11}$, Substance $P_{2-11}$, Substance MH-548 and Substance MH-648 of the formulae as indicated in Table 1 hereinbefore are especially preferred as the hair-growth-promoting active peptide. Preferred examples of the agent for controlling the absorption of the active peptide by the skin are cetanol and the aforesaid acidic, cross-linked acrylic acid, polymer available under a tradename "HIVIS WAKO-105". Preferred examples of the agent for promoting the penetration of the active peptide in the true skin layer are propylene glycol and di-ethyl sebacate.

In the compositions according to this invention, Substance P of the formula (II) or a Substance P-related peptide of the formula (I) as the active ingredient for promotion of the hair growth is dissolved or suspended in the excipient or vehicle present in the composition. The effective amount of the active peptide in the composition may be at such a concentration of the peptide in the composition exceeding 0.001% (weight/volume) and normally is in a range of from 0.005% to 0.05% and particularly of from 0.005% to 0.02% (weight/volume % , namely in term of the weight (in gramms) of the active peptide per 100 ml of the whole composition).

The active peptide of this invention is associated with the excipient or vehicle which comprises a material or a mixture of two or more materials conventionally used as the excipient in the hair-growth promotors or other cosmetic products of the prior art, and which may be, for example, water, ethanol, aqueous ethanol, a known creamy excipient or a known oily excipient.

In the hair-growth-promoting compositions of this invention, the higher aliphatic alcohol or the carboxyvinyl polymer as the agent for controlling the absorption of the active peptide may preferably be incorporated at a concentration of 0.5% to 5.0% (weight/volume) in the whole composition where said composition is prepared in the form of a lotion-like or tonic-like formulation, and it may preferably be incorporated at a concentration of 60% to 80% (weight/volume) in the composition where said composition is prepared in the form of a cream-like or ointment-like formulation.

In the hair-growth-promoting composition of the second aspect invention, the agent for promoting the penetration of the active peptide into the true skin is further incorporated therein for the purpose of facilitating that a quantity of the active peptide as released and supplied from the applied coat of the hair-growth-promoting composition to the skin surface penetrates and diffuses rapidly into the true skin layer.

The agent for promoting the penetration of the active peptide comprises a lower alkanol (except ethanol), a glycol, a mono-ester of a mono-carboxylic acid of 1 to 14 carbon atoms (especially an alkanoic acid of 9 to 14 carbon atoms) with an alcohol of 1 to 6 carbon atoms (especially an alkanol of 1 to 6 carbon atoms), or a di-ester of a di-carboxylic acid of 6 to 10 carbon atoms (especially an omega-carboxy-alkanoic acid or 6 to 10 carbon atoms) with an alcohol of 1 to 4 carbon atoms (especially with an alkanol of 1 to 4 carbon atoms), or a mixture of two or more of said lower alkanol, said glycol, said mono-ester and said di-ester. For example, the agent for promoting the penetration of the active peptide may preferably be a lower alkanol such as n-propanol and isopropanol; a glycol such as ethylene glycol, diethylene glycol, triethylene glycol, polyethylene glycol, propylene glycol and polypropylene glycol; a mono-ester such as isopropyl myristate, hexyl laurate, ethyl caproate; or a di-ester such as di-isopropyl adipate and di-ethyl sebacate. Amongst these examples of the agent for promoting the penetration of the active peptide, propylene glycol and di-ethyl sebacate are better for the active peptide.

The agent for promoting the penetration of the active peptide may suitably be incorporated at a concentration of 0.5% to 10%, preferably of 0.5% to 5% (weight/volume) in the composition of this invention. Addition of the agent for promoting the penetration of the active peptide in an increased proportion more than 5% (weight/volume) is not preferred, as it would unduly irritate the skin. The agent for promoting the penetration of the active peptide is also effective to make the skin more resilient and soft, so that it can play an additional useful role for the hair-growth-promoting effects of the active peptide of the formula (I) or (II). A suitable example of-the hair-growth-promoting formulation according to the first aspect invention comprises (i) the hair growth promoting active peptide; (ii) a higher aliphatic alcohol such as cetanol and lauryl alcohol or a carboxyvinyl polymer as the agent for controlling of the absorption of the active peptide; and (iii) water, ethanol or aqueous ethanol as the excipient. A suitable example of the hair-growth-promoting formulation according to the third aspect invention comprises (i) the hair growth promoting active peptide; (ii) cetanol, lauryl alcohol or the carboxyvinyl polymer as the agent for controlling the absorption of the active peptide; (iii) propylene glycol, or di-isopropyl adipate or di-ethyl sebacate as the agent for promoting the penetration of the active peptide; and (iv) water, ethanol or aqueous ethanol as the excipient.

When a hair growth promoting formulation of the first aspect invention is prepared mainly using cetanol or lauryl alcohol, glycerine (as a moistening agent) and water in association with the active peptide, the resulting formulation is in the form of an emulsifiable lotion. While, when a hair growth promoting formulation of the first aspect invention is prepared mainly using the carboxyvinyl polymer, ethanol and water, the resulting formulation is in the form of a gel-like tonic which rapidly liquefies upon being brought into contact with the skin and is thus readily applicable to the scalp skin. A cream-like formulation may be prepared in a known manner, for instance, by mixing together the active peptide, cetanol, lauryl alcohol, water, and a cationic, anionic or non-ionic surfactant to produce an oil-in-water (o/w) type dispersion or a water-in-oil (w/o) type dispersion. While, an ointment-like formulation may be prepared in a known manner, for instance, by mixing together the active peptide; cetanol, a lauryl alcohol or stearyl alcohol (as the higher aliphatic alcohol for controlling the absorption of the active peptide); and an oil-base excipient to produce an ointment.

Furthermore, the hair growth promoting compositions according to this invention can be given a good moisture-keeping property and a favorable or attractive feeling of use by incorporating thereinto a proportion of 3% to 5% (weight/volume) of glycerine, sodium lactate or 2-pyrrolidone-5-carboxylic acid sodium salt as such a moistening agent which prevent the drying of the skin horny layer unfavorable to the promotion of the hair growth and the prevention of the falling-out of the hair.

From the standpoint of view of the nutrition, the hair growth promoting compositions according to this invention may further contain one or more of a known vasodilator for capillary blood vessels such as acetylcholine; keratolytic such as salicylic acid; a promoter for the functions of the skin or a skin-irritant such as red-pepper; a moistening agent, a bacteriocide, perfuming agents, colorants, a thickening agent such as bleached bees-wax, and/or a surfactant such as sodium lauryl sulfate.

According to a particular, preferred embodiment according to the second aspect of this invention, there is provided a hair growth promoting composition comprising (a) Substance MH-548 as the active peptide for promoting the hair growth at a concentration of 0.01% to 0.05% (weight/volume) in the whole composition; (b) cetanol as the agent for controlling the absorption of the active peptide by the skin at a cetanol concentration of 1.5% to 5.0% (weight/volume) in the whole composition; and (d) propylene glycol as the agent for promoting the penetration of the active peptide into the true skin at a propylene glycol concentration of 0.5% to 5.0% (weight/ volume) in the whole composition; and (c) water or aqueous ethanol as the vehicle for the components of the composition; and the ratio of the amount of cetanol to the amount of Substance MH-548 present being in a range of 150:1 to 130:1 by weight.

According to a further particular, preferred embodiment according to the second aspect of this invention, there is also provided a hair growth promoting composition comprising (a) Substance MH-548 as the active peptide for promoting the hair growth at a concentration of 0.01% to 0.05% (weight/volume) in the whole composition; (b) an acidic, acrylic acid polymer having an average molecular weight of 1,000,000 to 3,000,000 as the agent for controlling the absorption of the active peptide at a concentration of said polymer of 0.5% to 5.0% (weight/volume) in the whole composition; (d) di-ethyl sebacate as the agent for promoting the penetration of the active peptide at a concentration of the di-ethyl sebacate of 0.5% to 5% (weight/volume) in the whole composition; (c) water or aqueous ethanol as the vehicle for the components of the composition, (e) sodium hydroxide in an amount just sufficient to make the whole composition neutral at pH 7.0. In the latter composition, the amount of the acrylic acid polymer may preferably be just sufficient to make the composition in a gel form at ambient temperature but make the composition into a sol when brought into contact with the scalp skin.

This invention is now illustrated with reference to the following Examples and Tests.

Example 1

Substance P (10 mg) (as the active ingredient for promotion of the hair growth) was mixed with 6 ml of ethanol (as an excipient) and 0.5 ml of glycerine (as a moistening agent), and the solution so formed was diluted with water to a total volume of 10 ml. The resultant aqueous solution was useful as a hair-growth-promoting formulation of the lotion-type containing 0.1% (w/v) of Substance P as the active ingredient.

Test No. 1

Different hair-growth-promoting formulations of the lotion-type each containing 0.1% (w/v) of an hair-growth-promoting active peptide as indicated in Table 2 below were prepared in the same manner as described in Example 1. Each formulation was applied repeatedly to the skin of mice to estimate the activity of the peptide for the promotion of the hair growth. The active peptide under test included Substance P, Substance $P_{6-11}$, Substance $P_{4-11}$ and MH-548 as representative examples of the active peptides listed in Table 1 hereinbefore. Four different hair-growth-promoting formulations containing the active peptide specified in Table 2, as well as a comparative formulation containing no active peptide (as a control) were tested for estimation of the activity of the peptide to promote the hair growth.

Table 2

| Sample designations of the hair growth promoting formulation under test | Compositions of the sample of the hair growth promoting formulation | |
|---|---|---|
| | Active peptide | Concentration of Active peptide (weight/volume, %) |
| Sample A (according to the first aspect invention) | Substance P | 0.1% |
| Sample B ( " ) | Substance $P_{6-11}$ | 0.1% |
| Sample C ( " ) | Substance $P_{4-11}$ | 0.1% |
| Sample D ( " ) | Substance MH-548 | 0.1% |
| Sample E (Control) | No addition | 0% |

Test Procedure

The hair was cut off from an area in the back of each of mice of $C_3H$ strain (male, 20-week-aged, 6 mice in each group). To the skin in the area of the mouse back where the hair had been removed, the hair-growth-promoting formulation was applied on the day next to the day of removal of the hair. The application of the formulation was made in 50 $\mu\ell$-portions four times per day and every days for the consecutive three weeks. One day after the last day of the application of the formulation, the hair as grown was removed from a particular region (1.5 cm x 2.5 cm) in the area of the mouse back where the formulation was repeatedly applied. The amount of the hair as removed was weighed (in mg) for estimation of the hair-growth-promoting effects of the formulation under test.

The removal of the hair was carried out according to such a procedure that the mouse was anaesthetized with ethyl ether and subsequently a shaving cream was applied to the area treated with the formulation, followed by cutting the hair off from said particular region by means of safety razor. The hair as removed was washed with water in a flask, and the hair was then collected by filtration, dried in air at 65 °C for 24 hours and left in a humid room for 24 hours before the amount of the hair was weighed. The weight of the hair so measured was assumed as the amount of the hair as grown.

The test results obtained are summarized in Table 3 below.

Table 3

| Hair growth promoting formulation under test | Amount of Hair as grown in Mouse Back (in mg) | | | | | | | |
|---|---|---|---|---|---|---|---|---|
| | Experiment No.1 | Experiment No.2 | Experiment No.3 | Experiment No.4 | Experiment No.5 | Experiment No.6 | Average value | Standard Deviation |
| Sample A (this invention) | 10.34 | 8.87 | 13.52 | 12.21 | 11.09 | 9.47 | 10.92 | 1.74 |
| Sample B ( " ) | 13.66 | 14.83 | 8.42 | 12.63 | 17.24 | 9.57 | 12.73 | 3.29 |
| Sample C ( " ) | 8.62 | 13.05 | 12.16 | 7.63 | 9.31 | 14.69 | 10.91 | 2.79 |
| Sample D ( " ) | 14.16 | 17.33 | 8.69 | 15.72 | 13.80 | 19.54 | 14.87 | 3.70 |
| Sample E (Control) | 6.37 | 5.24 | 8.61 | 4.28 | 7.05 | 6.12 | 6.28 | 1.49 |

Example 2

A mixture of 150 mg of cetanol (as the agent for controlling the absorption of the active peptide), 10 mg of bleached bees-wax (as a thickening agent), 50 mg of sodium lauryl sulfate (as a surfactant) and 7 ml of

water was heated at 95-100°C under well stirring until the mixture became a uniform and viscous liquid. This viscous liquid mixture was then admixed with such a solution which was prepared by adding 1 mg of Substance MH-548 identified in Table 1 hereinbefore (as the active peptide for promoting the hair growth) to a mixture of 0.5 ml of glycerine (as a moistening agent), 0.5 ml of propylene glycol ( as the agent for promoting the penetration of the active peptide) and 1 ml of water and then dispersing and solving Substance MH-548 into the latter mixture under supersonic actions. The admixture so obtained was well agitated and diluted with water to a total volume of 10 ml, affording a hair growth promoting formulation of the lotion-type according to the fifth aspect invention, which contained 0.015% (w/v) of Substance MH-548 as the active peptide.

Example 3

a solution of 400 mg of a carboxyvinyl polymer (the acrylic acid polymer commercially available under the tradename "HIVIS WAKO-105", from Japanese company, Wako Junyaku Kogyo Co., Ltd.; as the agent for controlling the absorption of the active peptide) in 35 ml of water was mixed with 3 ml of propylene glycol (as the agent for promoting the penetration of the active peptide) and further mixed with a solution of 7 mg of Substance P (as the active peptide for promoting the hair growth) in 35 ml of ethanol. The mixture so obtained was adjusted to pH 7.0 by addition of 1 N aqueous sodium hydroxide, so that said mixture became a gel. This gel was cooled to -10°C to liquefy. The resultant sol solution was diluted with water to a total volume of 70 ml and then allowed to be warmed to ambient temperature under stirring, to afford a hair growth promoting formulation of the gel-like tonic-type according to the second aspect invention, which contained 0.01% (w/v) of Substance P as the active peptide.

Example 4

A solution containing 100 mg of sodium lauryl sulfate (as a surfactant) and 400 mg of the carboxyvinyl polymer (the same acrylic acid polymer as used in Example 3 above, commercially available under a tradename "HIVIS WAKO-105", as the agent for controlling the absorption of the active peptide) dissolved in 30 ml of water was mixed with a solution of 7 mg of Substance MH-548 (as the active peptide for promoting the hair growth) and 0.5 ml of diethyl sebacate (as the agent for promoting the penetration of the active peptide) in 30 ml of ethanol and then was mixed with 3 ml of glycerine (as a moistenening agent). The mixture so obtained was adjusted to pH 7.0 by addition of 1 N aqueous sodium hydroxide, so that the mixture was gellified. The resultant gel was cooled to -10°C so that it was liquefied. The sol solution was diluted with water to a total volume of 70 ml and then allowed to be warmed to ambient temperature under stirring. A hair growth promoting formulation according to the second aspect of this invention was thus obtained, which was of the gel-like tonic type and contained 0.01% (w/v) of Substance MH-548 as the active peptide.

Example 5

A solution containing 1 mg of Substance MH-648 (as the active peptide) identified in Table 1 hereinbefore in a mixture of 6 ml of ethanol and 0.5 ml of propylene glycol (as the agent for promoting the penetration of the active peptide) was mixed with a solution of 500 mg of sodium lactate (as a moistening agent) and 0.2 ml of di-ethyl sebacate (as the agent for controlling the absorption of the active peptide) in 3 ml of water. The mixture so obtained was diluted with water to a total volume of 10 ml. A hair growth promoting formualtion of a liquid, tonic-type was thus obtained according to the second aspect of this invention, which contained 0.01% (w/v) of Substance MH-648 as the active peptide.

Example 6

The procedure of Example 1 was repeated but using 20 mg of cetanol in place of the 150 mg of cetanol without the propylene glycol. A hair growth promoting formulation of the lotion-type was thus prepared according to the first aspect of this invention, which contained 0.01% (w/v) of Substance MH-548 as the active peptide.

Example 7

The procedure of Example 1 was repeated but using 300 mg of cetanol in place of the 150 mg of

cetanol. A hair growth promoting formulation of the lotion-type was thus prepared according to the second aspect of this invention, which contained 0.015% (w/v) of Substance MH-548 as the active peptide.

Example 8

A solution of 400 mg of the carboxyvinyl polymer (same as the acrylic acid polymer as employed in Example 3 above) in 70 ml of water was mixed with a solution of 7 mg of Substance $P_{2-11}$ identified in Table 1 hereinbefore (as the active peptide for promoting the hair growth) in 5 ml of ethanol. The mixture obtained was adjusted to pH 7.0 by addition of 1 N aqueous sodium hydroxide, so that the mixture was gellified. The resultant gel was cooled to -10°C and thus liquefied to give a sol solution. The sol solution was diluted with water to a total volume of 70 ml and then allowed to be warmed to ambient temperature under stirring. A hair growth promoting formulation of a gel-like tonic type according to the first aspect of this invention was thus afforded which was in the form of a gel and contained 0.01% (w/v) of Substance $P_{2-11}$ as the active peptide.

Test No. 2

This test illustrates how much the application of the hair growth promoting formulation of this invention to the skin surface increased the temperature of the underlying true skin layer in mice.

Test (i)

We have presumed that the hair growth promoting effects of the active peptide, Substance P and Substance P-related peptide of the formula (II) used according to this invention can be obtained through such biological mechanism that the active peptide as applied to the skin surface is absorbed into the true skin layer and acts here as a vasodilator for the capillary blood vessels present here, resulting in an increase in the blood flow at the local site where the active peptide was applied to the skin surface.

For the purpose of confirming whether Substance P or Substance P-related peptide of the formula (II) is actually effective to increase the blood flow in the capillary blood vessels at a local area of the skin, we have conducted some experiments where the hair growth promoting formulation of this invention was applied to a local skin surface of monkies, and an increase in the temperature of the underlying true skin tissue below the local skin surface was measured, which should be considered to be invoked by such increase in the local blood flow at the local skin site owing to the vasodilating activity of Substance P or Substance P-related peptide of the formula (II).

Test procedure

Cynomolgus monkeys (Crab-eating macaques) (female, body weight 3.8-4.2 kg) were fixed each in a device for fixing the monkey. The hair was cut off from an area in the upper arms of each monkey. A probe of a thermometer for measurement of the temperature in a deep place (a thermometer commercially available from Telmo Co., Ltd., Japan) was sent into the hair-removed areas of each upper arm of the monkey, and the temperature in the true skin layer of the left and right upper arms of the monkey was measured before the administration of the active peptide for promoting the hair growth was made. After this, the probes of the thermometer were removed from the two arms, and to the hair-removed area of the skin surface of the right arm was applied 0.1 ml/2 $cm^2$ of a hair growth promoting formation of this invention which had been prepared in the same manner as described in Examples 2-8 above and which contained a hair growth promoting active peptide identified in Table 4 below. Concurrently, to the hair-removed area of the skin surface of the left arm of the monkey was applied 0.1 ml/2 $cm^2$ of a placebo preparation which was prepared in the same manner as in Examples 2-8 but without incorporating therein the active peptide used according to this invention. After the coats as applied of the hair growth promoting formulation and the placebo preparation were dried in air for 10 minutes, the probes of the thermometer each were set into the coat surfaces of the hair growth promoting formulation and of the placebo preparation as applied, and 20 minutes later (namely, at the end of totally 30 minutes after the application of the hair growth promoting formulation) the temperature in the true skin layer of the monkey arms was measured by means of the thermometers.

The test results obtained were assessed in the following way. Thus, the following parameters were evaluated.

$T_{RC}$--- Temperature in the untreated true skin of the right arm before the administration of the active

peptide;

$T_{LC}$--- The temperature in the untreated true skin in the left arm before the administration of the active peptide;

$T_{RT}$--- Temperature in the treated true skin in the right arm after the administration of the active peptide; and

$T_{LT}$--- Temperature in the placebo-treated true skin in the left arm after the administration of the placebo preparation.

Net effect ($T_E$) of increase in the temperature of the true skin due to the administration of the hair growth promoting formulation containing the active peptide was calculated according to the following equation:-

$$T_E = (T_{RT} - T_{LT}) - (T_{RC} - T_{LC}) = T_{RT} + T_{LC} - (T_{RC} + T_{LT})$$

The values of $T_E$ as calculated are shown in Table 4 below for estimation of the activity of the three active peptides, namely Substance P, Substance MH-548 and Substance MH-648.

Table 4

Effect of the hair growth promoting formulations under test to increase the temperature in the true skin

| | $T_E$ (°C) | | |
|---|---|---|---|
| | Active Peptide | | |
| Test Sample | Substance P | Substance MH-548 | Substance MH-648 |
| Formulation containing active peptide + cetanol + propylene glycol and as prepared by the same procedure as described in Example 2 (according to the fifth aspect of this invention) | 0.1 | 0.4 | 0.2 |
| Formulation containing active peptide + carboxyvinyl polymer + propylene glycol and as prepared by the same procedure as described in Example 3 (according to the fifth aspect of this invention) | 0.4 | 0.7 | 0.5 |
| Formulation containing active peptide + carboxyvinyl polymer + di-ethyl sebacate and as prepared by the same procedure as described in Example 4 (according to the fifth aspect of this invention) | 0.4 | 0.9 | 0.6 |
| Formulation containing active peptide + di-ethyl sebacate + propylene glycol and as prepared by the same procedure as described in Example 5 (according to the fifth aspect of this invention) | 0.3 | 0.4 | 0.3 |
| Formulation containing active peptide + cetanol and as prepared by the same procedure as described in Example 6 (according to the fourth aspect of this invention) | 0.1 | 0.2 | 0.1 |
| Formulation containing active peptide + cetanol + propylene glycol and as prepared by the same procedure as described in Example 7 (according to the fifth aspect of this invention) | 0.1 | - | - |

Cont'd......

| | | | |
|---|---|---|---|
| Formulation containing active peptide + carboxyvinyl polymer and as prepared by the same procedure as described in Example 8 (according to the fourth aspect of this invention) | - | - | 0.1 |

From the results shown in Table 4 above, it is seen that the formulations as prepared by the procedures of Examples 3 and 4 exhibit a much enhanced effect of increasing the temperature in the true skin, as compared to the other formulations under test, revealing that such a formulation of the gel-like tonic type containing the carboxyvinyl polymer as the agent for controlling the absorption of the active peptide by the skin is better than the other types of the formulations under test. Besides, it is seen that the incorporation of propylene glycol into the formulation, either of the tonic type or of the lotion type, can enhance the penetration of the active peptide through the true skin and hence enhance the effects of the active peptide to increase the temperature in the true skin. On the other hand, we have obtained some another test results which show that when the proportion of cetanol to the active peptide in the formulation is too much, the release of the active peptide from the applied coat of the formulation into the skin can unduly be suppressed so that no effect of increasing the temperature in the true skin is obtained even after lapse of 30 minutes from the administration of the active peptide. This has revealed that the proportion of cetanol to the active peptide in the hair growth promoting formulation of this invention should be chosen appropriately. For instance, we have found that the proportion of cetanol as incorporated is desirably 1.5% (w/v) or thereabout with such a formulation which has been prepared by the same procedure as described in Example 1 and contained 0.01% (w/v) of Substance MH-548 as the active peptide. In general, it is preferred that the ratio of the weight of cetanol as incorporated to the weight of Substance MH-548 in the hair growth promoting formualtion of this invention should be in a range of 150:1 to 180:1 for a proper rate of the release of the active peptide from the applied coat of the formulation.

Furthermore, it is seen that amongst the different active peptides tested, Substance MH-548 is most effective to increase the temperature in the true skin as treated.

Test (ii)

For the purpose of examining the functions of different ester compounds available as the agent for promoting the penetration of the active peptide in the hair growth promoting formulation according to the second aspect of this invention, we have conducted some experiments which to show how much the effect of the formulation for increasing the temperature in the true skin varied with the nature and concentration of said different ester compounds incorporated as the agent for promoting the penetration of the active peptide.

Thus, 14 different hair growth promoting formulations of the gel-like tonic type and containing Substance MH-548 as the active peptide were prepared by repeating the procedure of Example 3 above, except that Substance P was replaced by Substance MH-548, that 100 mg of sodium lauryl sulfate was additionally incorporated into each formulation, and that the polypropylene glycol was replaced by isopropyl myristate, di-isopropyl adipate or di-ethyl sebacate as incorporated at a varying concentration of 0.1 to 10% (w/v) in the formulation. These fourteen formulations prepared were tested in the same manner as in Test (i) above, for estimation of their effects of increasing the temperature in the true skin of the arm of the monkey, in term of the value of "$T_E$" as defined hereinbefore. The test results obtained are summarized in Table 5 below. From the test results of Table 5, it is clear that di-ethyl sebacate at a concentration of 0.5% (w/v) or more exhibited a better effect than isopropyl myristate and di-isopropyl adipate.

Table 5

Influence of different esters as the agent for promoting the penetration of the active peptide on the effect of the hair growth promoting formulation to increase the temperature in the true skin

| Nature of esters tested as the agent for promoting the active peptide penetration | $T_E$ (°C) Concentration (%, w/v) of the agent for promoting the active peptide penetration | | | | |
|---|---|---|---|---|---|
| | 0.1% | 0.5% | 1% | 5% | 10% |
| Isopropyl myristate | - | 0.2 | 0.4 | 0.5 | 0.3 |
| Di-isopropyl adipate | 0.1 | 0.4 | 0.3 | 0.3 | 0.4 |
| Di-ethyl sebacate | 0.1 | 0.7 | 0.7 | 0.5 | 0.8 |

Test No. 3

Eight formulations were prepared, including three hair growth promoting formulations according to the second aspect of this invention and containing Substance MH-548 as the active peptide, as well as five "comparative" formulations, either containing an active compound known as the hair growth promotor or containing none of the hair growth promoting compounds. The compositions of the eight formulations prepared are shown in Table 6 below. Some experiments for promoting the hair were carried out by applying each formulation onto the skin of the mouse.

Table 6

| Designation of formulation under test | Composition of the formulations tested for promotion of the hair growth |
|---|---|
| Sample F (according to this invention) | Formulation as prepared in Example 2 (in the form of a lotion containing 0.01% (w/v) of Substance MH-548 as the active peptide + cetanol + propylene glycol) |
| Sample G (according to this invention) | Formulation as prepared similarly to Example 2 with the amount of Substance MH-548 being reduced to 0.1 mg (in the form of a lotion containing 0.001% (w/v) of MH-548 + cetanol + propylene glycol) |
| Sample H (according to this invention) | Formulation as prepared in Example 4 (in the form of a gel-like tonic containing 0.01% (w/v) of Substance MH-548 + the carboxyvinyl polymer + di-ethyl sebacate) |
| Sample I (Comparative) | Formulation as prepared similarly to Example 2 but with omitting the active peptide (as the lotion base) |
| Sample J (Comparative) | Formulation as prepared similarly to Example 4 but with omitting the active peptide (as the gel-like tonic base) |
| Sample K (Comparative) | A certain known, commercially available hair growth promoting formulation (containing 5% (w/v) of an acetyl chloline-like active substance) |
| Sample L (Comparative) | Simple water |
| Sample M (Comparative) | A solution as obtained by diluting the Sample K hair growth promoting formulation with a mixture of ethanol and water (1:1) to a volume of 10-folds the original volume of said formulation |

Test procedure

The hair was shaved from an area in the back of mice of $C_3H$ strain (male, 20-weeks-aged, six mice in each group). On the next day, the formulation under test was applied to the shaved area and the application

of the formulation was made twice per day and every day for the consecutive 3 weeks at a rate of application of 50 $\mu \ell$/4 $cm^2$ each time to effect the treatment for promotion of the hair growth. During the treatment period, one mouse died in the group of mice treated with Sample K, probably due to any adverse effect of uncertained substance present in Sample K, so that the hair growth promoting treatment was finished only with five mice in the group of mice treated with Sample K. Five photographs in each set for one formulation under test were taken of the condition of the hair as grown in the treated area of the mouse back on the day just before the day of shaving, at a time just after the shaving (before the application of the formulation under test), on the day after lapse of one week from the first application of the formulation, on the day after lapse of two weeks from the first application of the formulation and on the day after lapse of three weeks from the first application of the formulation, respectively. The photographs as taken were shown to a first panel consisting of 8 male members and a second panel consisting of 8 female members, and their replies were obtained by the questionnaire method to indicate which one of the photographs was better or worse than the another in showing favorable condition of the hair growth, so that the ranking of the hair growth-promoting efficacy of the formulations under test could be decided. The members of the two panels who answered the questionnaire were chosen at random from a class of many persons. The photographs of each set were shown to each of the panel members in irregular order and the questionnaire was conducted to obtain their replies to indicate the ranking of the photographed conditions of the hair growth in the order of 1, 2, 3 ... 8 from better to worse. On another day, a second questionnaire was made to the same panel members and in the same manner as in the first questionnaire, but in such a way that the different set of photographs from the first set was shown to the same person.

The test results obtained through the above questionnaires were assessed according to a method for the analysis of variance as described in a Japanese medicinal journal "Sa-i-shin I-gaku" Vol. 29, pages 1000-1006 (1974).

It was concluded that the sequence of the higher efficacy of the hair growth promoting activity of the formulations under test was in the order of Sample H>Sample F>Sample J >Sample K>Sample M>Sample L>Sample G>Sample I. This reveals that Samples H and F corresponding to the hair growth promoting formulations shown in the Examples 2 and 4 remarkedly improved the amount of the hair grown, as compared with the groups of mice treated with the Sample I (the lotion base containing no active peptide) and with Sample J (the tonic base containing no active peptide) and with the group of mice untreated (i.e., treated with simple water only), and also that the Samples H and F could resulted in an improved hair growth over the commercially available known hair growth promoter tested as Samples K and M.

**Claims**

**1.** A hair growth promoting composition comprising (a) an effective amount of at least one of Substance P and a Substance P-related peptide of the formula (I)

X-Phe-Phe-Gly-Leu-Met-$NH_2$ (I)

wherein Phe denotes the phenylalanine residue, Gly the glycine residue, Leu the leucine residue, Met the methionine residue, and X denotes the pyro-glutamic acid residue or a peptide composed of 2 to 5 amino acid residues or a nucleoside, or an acid addition salt thereof as the active ingredient for promotion of the hair growth, and (b) at least one of higher aliphatic alcohols and carboxyvinyl polymers which are effective as an agent for controlling the absorption of the active peptide by the skin, in association with (c) a known vehicle or excipient for the active peptide (a) and the agent (b) for controlling the absorption of the active peptide.

**2.** The composition as claimed in Claim 1 in which the amount of (a) Substance P or the Substance P-related peptide of the formula (I) as the active peptide for promoting the hair growth is at a concentration of 0.005% to 0.05% (weight/volume) in the whole composition; and the amount of (b) the agent for controlling the absorption of the active peptide is at a concentration of 0.5% to 5.0% (weight/volume) in the whole composition.

**3.** A hair growth promoting composition comprising (a) an effective amount of at least one of Substance P and a Substance P-related peptide of the formula (I)

X-Phe-Phe-Gly-Leu-Met-$NH_2$ (I)

wherein Phe denotes the phenylalanine residue, Gly the glycine residue, Leu the leucine residue, Met the methionine residue, and X denotes the pyro-glutamic acid residue or a peptide composed of 2 to 5 amino acid residues or a nucleoside, or an acid addition salt thereof as the active ingredient for promotion of the hair growth, (b) at least one of higher aliphatic alcohols and carboxyvinyl polymers which are effective as an agent for controlling the absorption of the active peptide by the skin, and (d) at least one of lower alkanols, glycols, mono-esters of a mono-carboxylic acid of 9 to 14 carbon atoms with an alkanol of 1 to 6 carbon atoms, and di-esters of a di-carboxylic acid of 6 to 10 carbon atoms with an alkanol of 1 to 4 carbon atoms which are effective as an agent for promoting the penetration of the active peptide into the true skin, in association with (c) a known vehicle or excipient for the active peptide (a) and the agent (b) for controlling the absorption of the active peptide as well as (c) the agent for promoting the penetration of the active peptide.

4. The composition as claimed in claim 3 in which the amount of (a) Substance P or the Substance P-related peptide as the active peptide for promoting the hair growth is at a concentration of 0.005% to 0.05% (weight/volume) in the whole composition; the amount of (b) the agent for controlling the absorption of the active peptide is at a concentration of 0.5% to 5.0% (weight/volume) in the whole composition; and the amount of (d) the agent for promoting the penetration of the active peptide is at a concentration of 0.5% to 10.0% (weight/volume) in the whole composition.

5. A hair growth promoting composition comprising (a) Substance MH-548, Substance MH-548 being represented by the formula

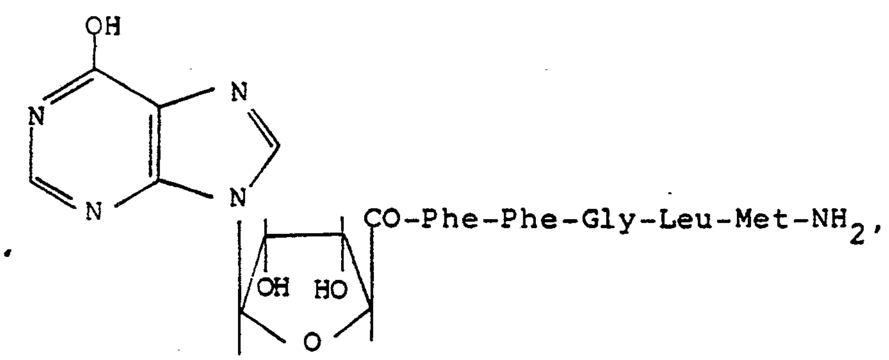

as the active peptide for promoting the hair growth at a concentration of 0.01% to 0.05% (weight/volume) in the whole composition; (b) cetanol as the agent for controlling the absorption of the active peptide by the skin at a cetanol concentration of 1.5% to 5.0% (weight/volume) in the whole composition; and (d) propylene glycol as the agent for promoting the penetration of the active peptide into the true skin at a propylene glycol concentration of 0.5% to 5.0% (weight/volume) in the whole composition; and (c) water or aqueous ethanol as the vehicle for the components of the composition; and the ratio of the amount of cetanol to the amount of Substance MH-548 present being in a range of 150:1 to 130:1 by weight.

6. A hair growth promoting composition comprising (a) Substance MH-548 as the active peptide for promoting the hair growth at a concentration of 0.01% to 0.05% (weight/ volume) in the whole composition; (b) an acidic, acrylic acid polymer having an average molecular weight of 1,000,000 to 3,000,000 as the agent for controlling the absorption of the active peptide at a concentration of said polymer of 0.5% to 5.0% (weight/volume) in the whole composition; (d) di-ethyl sebacate as the agent for promoting the penetration of the active peptide at a concentration of the di-ethyl sebacate of 0.5% to 5% (weight/ volume) in the whole composition; (c) water or aqueous ethanol as the vehicle for the components of the composition, (e) sodium hydroxide in an amount just sufficient to make the whole composition neutral at pH 7.0.

7. The composition as claimed in Claim 6 in which the amount of the acrylic acid polymer is just sufficient to make the composition in a gel form at ambient temperature but make the composition in a sol form when brought into contact with the scalp skin.

## Revendications

1. Composition stimulant la pousse des cheveux, comprenant (a) une quantité efficace d'au moins un composé choisi parmi la substance P et un peptide apparenté à une substance P de formule (I) :

   X-Phe-Phe-Gly-Leu-Met-$NH_2$ (I)

   dans laquelle Phe représente le résidu phénylalanine, Gly représente le résidu glycine, Leu représente le résidu leucine, Met représente le résidu méthionine, et X représente le résidu dérivé d'acide pyroglutamique (pGlu), un peptide comprenant 2 à 5 résidus dérivés d'aminoacide, un nucléoside, ou un sel d'addition avec un acide de celui-ci en tant qu'ingrédient actif stimulant la pousse des cheveux, et (b) au moins un composé choisi parmi les alcools aliphatiques supérieurs et les polymères carboxvinyliques efficaces en tant qu'agent de régulation de l'absorption du peptide actif par la peau, associés avec (c) un véhicule ou un excipient adapté au peptide actif (a) et à l'agent (b) de régulation de l'absorption du peptide actif.

2. Composition selon la revendication 1, dans laquelle la quantité (a) de la substance P ou du peptide apparenté à la substance P de formule (I) en tant que peptide actif stimulant la pousse des cheveux, correspond à une concentration de 0,005 % à 0,05 % (poids/volume) dans la composition complète ; et dans laquelle la quantité (b) de l'agent de régulation de l'absorption du peptide actif, correspond à une concentration de 0,5 % à 5,0 % (poids/volume) dans la composition complète.

3. Composition stimulant la pousse des cheveux, comprenant (a) une quantité efficace d'au moins un composé choisi parmi la substance P et un peptide apparenté à la substance P, de formule (I) :

   X-Phe-Phe-Gly-Leu-Met-$NH_2$ (I)

   dans laquelle Phe représente le résidu phénylalanine, Gly représente le résidu glycine, Leu représente le résidu leucine, Met représente le résidu méthionine, et X représente le résidu dérivé d'acide pyroglutamique, un peptide composé de 2 à 5 résidus dérivés d'aminoacide, un nucléoside, ou un sel d'addition avec un acide de celui-ci en tant qu'ingrédient actif stimulant la pousse des cheveux, (b) au moins un composé choisi parmi les alcools aliphatiques supérieurs et les polymères carboxyvinyliques efficaces en tant qu'agent de régulation de l'absorption du peptide actif par la peau, et (d) au moins un composé choisi parmi les alcanols inférieurs, les glycols, les monoesters d'acide monocarboxylique comportant de 9 à 14 atomes de carbone, avec un alcanol comportant de 1 à 6 atomes de carbone, et les diesters d'acide dicarboxylique comportant de 6 à 10 atomes de carbone, avec un alcanol comportant de 1 à 4 atomes de carbone, efficaces en tant qu'agent de stimulation de la pénétration du peptide actif dans le derme, associés avec (c) un véhicule ou un excipient connu adapté au peptide actif (a) et à l'agent (b) de régulation de l'absorption du peptide actif, ainsi qu'à l'agent (d) de stimulation de la pénétration du peptide actif.

4. Composition selon la revendication 3, dans laquelle la quantité (a) de substance P ou du peptide apparenté à la substance P en tant que peptide actif stimulant la pousse des cheveux, correspond à une concentration de 0,005 % à 0,05 % (poids/volume) dans la composition complète ; dans laquelle la quantité (b) de l'agent de régulation de l'absorption du peptide actif, correspond à une concentration de 0,5 % à 5,0 % (poids/volume) dans la composition complète ; dans laquelle la quantité (d) de l'agent de stimulation de la pénétration du peptide actif, correspond à une concentration de 0,5 % à 10,0 % (poids/volume) dans la composition complète.

5. Composition stimulant la pousse des cheveux, comprenant (a) la substance MH-548 représentée par la formule :

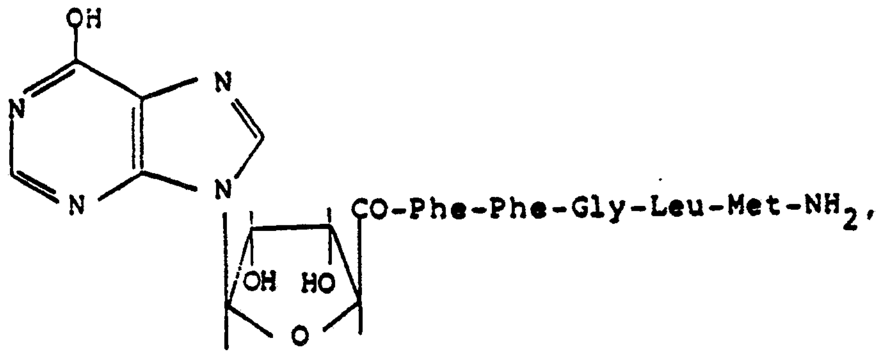

en tant que peptide actif stimulant la pousse des cheveux, à une concentration de 0,01 % à 0,05 % (poids/volume) dans la composition complète ; (b) du cétanol en tant qu'agent de régulation de l'absorption du peptide actif par la peau, à une concentration en cétanol de 1,5 % à 5,0 % (poids/volume) dans la composition complète ; et (d) du propylène glycol en tant qu'agent de stimulation de la pénétration du peptide actif dans le derme, à une concentration en propylène glycol de 0,5 % à 5,0 % (poids/volume) dans la composition complète ; et (c) de l'eau ou de l'éthanol aqueux en tant que véhicule des composants de la composition ; et dans laquelle le rapport de la quantité de cétanol à la quantité de substance MH-548 présents, est de 150:1 à 130:1 en poids.

**6.** Composition stimulant la pousse des cheveux, comprenant (a) la substance MH-548 en tant que peptide actif stimulant la pousse des cheveux, à une concentration de 0,01 % à 0,05 % (poids/volume) dans la composition complète ; (b) un polymère acide dérivé de l'acide acrylique, ayant un poids moléculaire moyen de 1 000 000 à 3 000 000, en tant qu'agent de stimulation de l'absorption du peptide actif, à une concentration de ce polymère, de 0,5 % à 5,0 % (poids/volume) dans la composition complète ; (d) du sébaçate de diéthyle en tant qu'agent de stimulation de la pénétration du peptide actif, à une concentration de sébaçate de diéthyle, de 0,5 % à 5 % (poids/volume) dans la composition complète ; (c) de l'eau ou de l'éthanol aqueux en tant que véhicule pour les composants de la composition, et (e) de l'hydroxyde de sodium selon une quantité juste suffisante pour neutraliser la composition complète au pH de 7,0.

**7.** Composition selon la revendication 6, dans laquelle la quantité de polymère dérivé d'acide acrylique, est juste suffisante pour gélifier la composition à la température ambiante, mais pour convertir la composition sous la forme d'un sol lorsqu'elle est mise en contact avec le cuir chevelu.

**Patentansprüche**

**1.** Mittel zum Stimulieren des Haarwuchses, das enthält: (a) als aktiven Bestandteil zur Stimulierung des Haarwuchses eine wirksame Menge von wenigens einem der nachfolgenden Stoffe, nämlich Substance P oder ein Substance P verwandtes Peptid mit der Formel (I)

$X\text{-}Phe\text{-}Phe\text{-}Gly\text{-}Leu\text{-}Met\text{-}NH_2$ (I),

wobei Phe den Phenylalanin-Rest, Gly den Glyzin-Rest, Leu den Leucin-Rest, Met den Methionin-Rest und X den Pyroglutaminsäure-Rest oder ein aus 2 bis 5 Aminosäureresten oder Nucleosiden bestehendes Peptid bezeichnet, oder ein durch Säurezugabe erhaltenes Salz hiervon, sowie (b) als Wirkstoff zur Kontrolle der Absorption des aktiven Peptids durch die Haut wenigstens einen der nachfolgenden Stoffe, nämlich einen höheren aliphatischen Alkohol oder ein CarboxyvinylPolymer, zusammen mit (c) einem bekannten Vehikel oder Exzipienten für das aktive Peptid (a) und den Wirkstoff (b) zur Kontrolle der Absorption des aktiven Peptids.

**2.** Mittel nach Anspruch 1, bei dem die Menge von (a) , der Substance P oder dem Substance P verwandten Peptid mit der Formel (I) als aktivem Peptid zur Stimulation des Haarwuchses bei einer Konzentration zwischen 0,005% und 0,05% (Gewicht/Volumen) in der gesamten Zusammensetzung vorliegt und bei der die Menge von (b) ,dem Wirkstoff zum Kontrollieren der Absorption des aktiven

Peptids in einer Konzentration zwischen 0,5% bis 5,0% (Gewicht/Volumen), in der Gesamtzusammensetzung vorliegt.

3. Mittel zum Stimulieren des Haarwuchses, das enthält: (a) als aktiven Bestandteil zur Stimulierung des Haarwuchses eine wirksame Menge von wenigens einem der nachfolgenden Stoffe, nämlich Substance P oder ein Substance P verwandtes Peptid mit der Formel (I)

X-Phe-Phe-Gly-Leu-Met-$NH_2$ (I),

wobei Phe den Phenylalanin-Rest, Gly den Glyzin-Rest, Leu den Leucin-Rest, Met den Methionin-Rest und X den Pyroglutaminsäure-Rest oder ein aus 2 bis 5 Aminosäureresten oder Nucleosiden bestehendes Peptid bezeichnet, oder ein durch Säurezugabe erhaltenes Salz hiervon, sowie (b) als Wirkstoff zur Kontrolle der Absorption des aktiven Peptids durch die Haut wenigstens einen der nachfolgenden Stoffe, nämlich einen höheren aliphatischen Alkohol oder ein CarboxyvinylPolymer, und (d) zum Stimulieren des Eindringens des aktiven Peptids in die Lederhaut wenigstens einen der nachfolgenden Stoffe, nämlich einen niedrigen Alkanol, Glycol, Monoester aus einer Mono-Carboxylsäure aus 9 bis 14 Kohlenstoffatomen und einem Alkanol aus 1 bis 6 Kohlenstoffatomen oder einen Diester aus einer Dicarboxylsäure mit 6 bis 10 Kohlenstoffatomen und einem Alkanol aus 1 bis 4 Kohlenstoffatomen, zusammen mit (c) einem bekannten Vehikel Oder Exzipienten für das aktive Peptid (a) und den Wirkstoff (b) zur Kontrolle der Absorption des aktiven Peptids ebenso wie des Wirkstoffs (d) zur Stimulierung des Eindrigens des aktiven Peptids.

4. Mittel nach Anspruch 3, bei dem die Menge von (a), der Substance P oder dem Substance P verwandten Peptid als aktivem Peptid zur Stimulation des Haarwuchses bei einer Konzentration zwischen 0,005% und 0,05% (Gewicht/Volumen) in der gesamten Zusammensetzung vorliegt; die Menge von (b) dem wirkstoff zum Kontrollieren der Absorption des aktiven Peptids in einer Konzentration zwischen 0,5% und 5,0% (Gewicht/Volumen) in der gesamten Zusammensetzung vorliegt; und die Menge von (b) dem Wirkstoff zum Stimulieren des Eindringens des aktiven Peptids in einer Konzentration zwischen 0,5% und 10% (Gewicht/Volumen) in der gesamten Zusammensetzung vorliegt.

5. Mittel zum Stimulieren des Haarwuchses, das enthält: (a) als aktives Peptid zur Stimulierung des Haarwuchses wenigestens einen der nachfolgenden Stoffe, nämlich Substance MH-548, Substance MH-548 gemäß der Formel

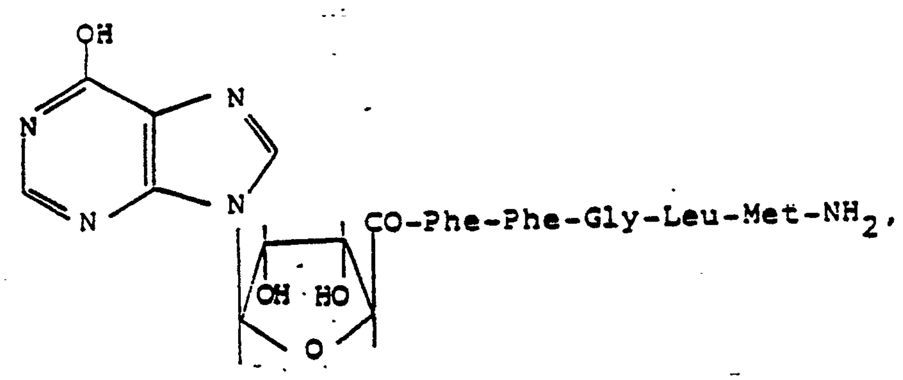

in einer Konzentration zwischen 0,01% und 0,05% (Gewicht/ Volumen) in der gesamten Zusammensetzung; (b) als Wirkstoff zum Kontrollieren der Absorption des aktiven Peptids durch die Haut Cetanol mit einer Cetanolkonzentration zwischen 1,5% und 5% (Gewicht/Volumen) in der gesamten Zusammensetzung; und (d) als Wirkstoff zum Stimulieren des Eindringens des aktiven Peptids in die Lederhaut Propylenglycol bei einer Propylenglycolkonzentration zwischen 0,5% und 5% (Gewicht/Volumen) in der gesamten Zusammensetzung; und (c) als Vehikel für die Komponenten der Zusammensetzung Wasser oder wässriger Ethanol; wobei das Verhältnis der Menge von Cetanol zu der Menge von vorhandenem Substance MH-548 im Bereich zwischen 150:1 und 130:1, bezogen auf das Gewicht, beträgt.

6. Mittel zum Stimulieren des Haarwuchses, das enthält: (a) als aktives Peptid zur Stimulierung des

Haarwuchses Substance MH-548 bei einer Konzentration zwischen 0,01% und 0,05% (Gewicht/Volumen) in der gesamten Zusammensetzung; (b) als Wirkstoff zur Kontrolle der Absorption des aktiven Peptids ein saures Acrylsäurepolymer mit einem mittleren Molekulargewicht von 1000000 bis 3000000 bei einer Konzentration des Polymers zwischen 0,5% und 5,0% (Gewicht/Volumen) in der gesamten Zusammensetzung; (d) als Wirkstoff zur Stimulierung des Eindringens des aktiven Peptids Diethylsebacat bei einer Konzentration des Diethylsebacats zwischen 0,5% und 5% (Gewicht/Volumen) in der gesamten Zusammensetzung; (c) als Vehikel für die Komponenten der Zusammensetzung Wasser oder wässrigen Ethanol und (e) Natriumhydroxid in einer Menge, die gerade ausreicht, um die gesamte Zusammensetzung bei pH 7,0 neutral zu machen.

**7.** Mittel nach Anspruch 6, bei dem die Menge des Acrylsäurepolymers gerade ausreicht, um die Zusammensetzung bei Umgebungstemperatur in eine Gelform, jedoch die Zusammensetzung in eine flüssige Form zu bringen, wenn sie mit der Kopfhaut in Berührung gebracht wird.